# EUROPEAN PATENT APPLICATION

(11) **EP 1 749 817 A1**
(43) Date of publication of application: **07.02.2007**
(21) Application number: 05741421.1
(22) Date of filing: 23.05.2005
(51) Int. Cl.: C07D 209/46, C07D 209/64, C07D 209/70, C07D 405/04, C07D 491/048, C07D 491/056, A61K 31/407, A61K 31/437, A61K 31/454, A61K 31/496, A61P 25/04, A61P 29/00

(54) **NEUROGENIC PAIN CONTROL AGENT COMPOSITION**

(30) Priority: 24.05.2004 JP 2004153206
(71) Applicant: Maruishi Pharmaceutical Co., Ltd., Osaka-shi, Osaka 541-0044 (JP)
(72) Inventor: YOSHIMURA, Masakazu, Kobe-shi, Hyogo 6570855 (JP); KANAMITSU, Norimasa, 6650841 (JP); ITSUJI, Yutaka, Osaka-shi Osaka 5550012 (JP); OSAKI, Takashi, 5730157 (JP); KAWASHIMA, Motoko, 5750061 (JP)
(74) Representative: von Kreisler, Alek
(86) International application number: PCT/JP2005/009361
(87) International publication number: WO 2005/113501

(57) **Abstract**

The instant application provides a pharmaceutical composition for controlling neuropathic pain, which comprises a compound of formula: 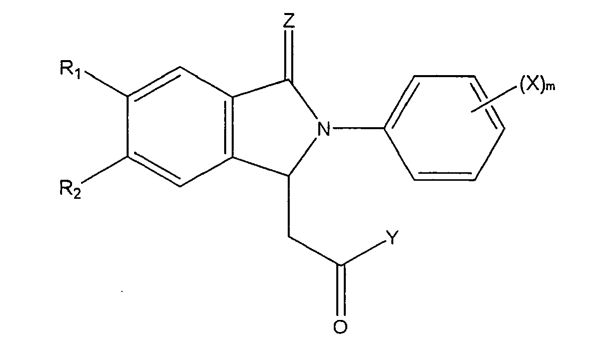 or a salt thereof.

## Description

### FIELD OF THE INVENTION

The present invention relates to a novel pharmaceutical use of isoindoline derivatives for controlling neuropathic pain. The invention also provides novel isoindoline derivatives.

### RELATED ART

Many compounds having isoindoline structure have been reported to have effects on central nerves system. Most of those reports aimed for developing tranquilizers, antispasmodics or anxiolytics (Japanese Patent Application Laid Open Nos. 47-12322 and 58-189163). Heretofore, no isoindoline derivative effective for treating all kinds of pains has been reported.

Neuropathic pain is a pain initiated or caused by a primary lesion or dysfunction in the nervous system and not by stimulation in a peripheral sensory organ. Examples of neuropathic pains, acute pain caused by herpes zoster, post herpetic neuralgia, painful neuropathy of diabetes and cancer pain are known. Severe pain episodes extensively deteriorate the patient's Quality of Life, for example, those patients tend to be suffered from depression

In general, "analgesics" are divided in two groups: anti-inflammatory analgesics such as aspirin and narcotic analgesics such as morphine. However, those analgesics have been revealed to be less effective for treating neuropathic pain. In these days, medicaments like anticonvulsant, antidepressant and anti anxiolytic are used for controlling neuropathic pain. They can provide temporal relief from the pain but cannot be used for a long period due to their adverse side effects. Accordingly, a pharmaceutical composition effective for treating neuropathic pain and induce less or no side effects have been desired in the clinical field.

Gabapentin, which is believed useful for treating neuropathic pain and clinically used in Europe, had initially been developed as anticonvulsant and the effect of the same on neuropathic pain had been found upon its clinical use. Gabapentin have been revealed effective for treating most of neuropathic pains. Some side effects include vertigo and lightheadedness reported hereto, but no severe case has been reported. In general, gabapentin has a slow onset of action. In general, the analgesic action onsets after 1-2 hour of oral administration or after several administration. In order to control acute pain occurs suddenly, an analgesic whose onset of action occurs soon after the administration is desired.

As discussed above, no satisfying rapid-acting compound for controlling neuropathic pain with less side effects has been obtained heretofore.

### SUMMARY OF THE INVENTION

An object of the invention is to provide a pharmaceutical composition for suppressing neuropathic pain. Further object of the present invention is to provide novel isoindoline derivatives.

### METHOD FOR SOLVING THE PROBLEM

The present invention provides a composition for controlling neuropathic pain, comprising a compound represented by formula (I): 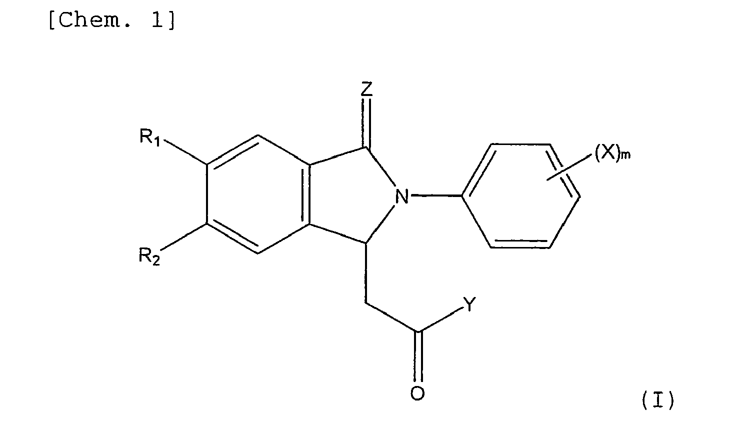 wherein R₁ and R₂ may be the same or different and represent C1-6 alkyl groups, or R₁ and R₂ taken together form 6-membered condensed ring having conjugated double bonds, or a moiety of -O-CH₂-O-, -CH₂-CH₂-CH₂- or -CH₂-O-CH₂-
X is halogen or C1-6 alkoxy, or taken together with the phenyl moiety to which the X is attached a group: 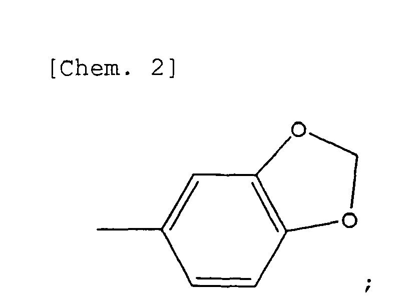 m is an integer of 0-2, provided that when X is C1-6 alkoxy, m is 0 or 1;
Y is 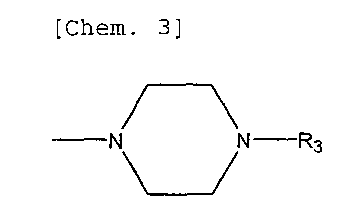 wherein R₃ is linear or branched C1-6 alkyl, linear or branched C3-6 alkenyl, linear or branched C3-6 alkynyl, C5-6 cycloalkyl, 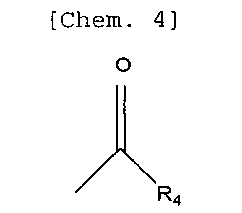 wherein R₄ is C1-4 alkyl, or 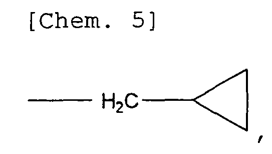 or 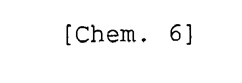 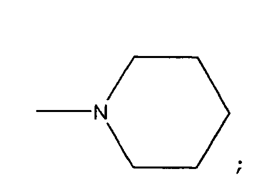 and
Z is oxygen or sulfur
or a salt thereof.

The present invention further provides a method for controlling neuropathic pain which comprises administering an effective amount of the compound of formula (I) to a patient in need of pain control.

The present invention also provides use of the compound of formula (I) for manufacturing a pharmaceutical composition for controlling neuropathic pain.

A part of the compounds which may be comprised in the composition of the invention are novel and accordingly, the instant invention also provides those novel compounds. The novel compounds of the invention are as follows:

| | |
|---|---|
| 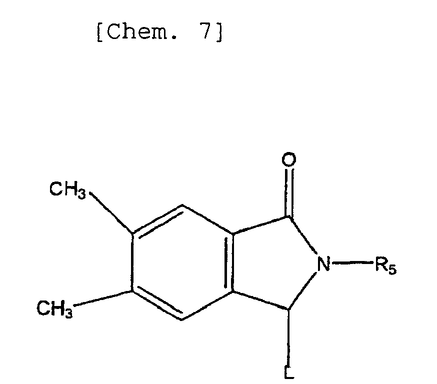 | |

| R5 | L |
|---|---|
| 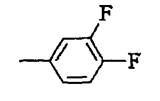 | 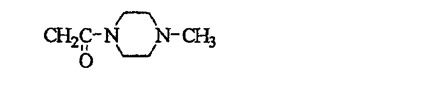 |
| 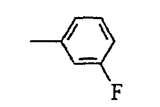 | 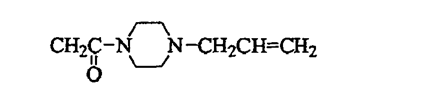 |
| 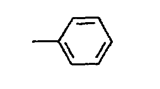 | 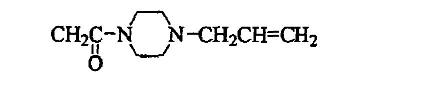 |
| 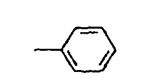 | 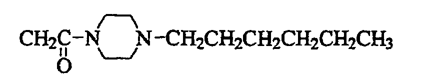 |
| 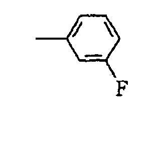 | 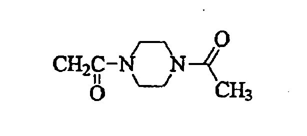 |

| | | |
|---|---|---|
| 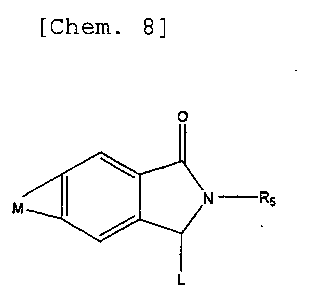 | | |

| M | R5 | L |
|---|---|---|
| CH₂CH₂CH₂ | 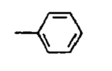 | 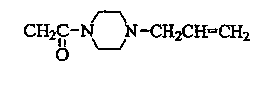 |
| CH₂CH₂CH₂ | 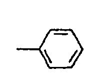 | 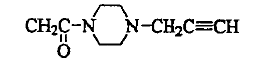 |
| CH₂CH₂CH₂ | 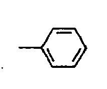 | 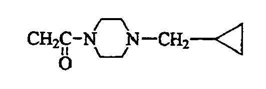 |
| OCH₂O | 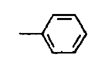 | 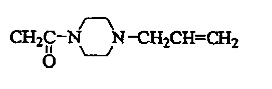 |
| CH₂OCH₂ | 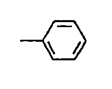 | 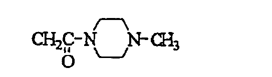 |
| CH₂OCH₂ | 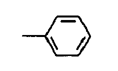 | 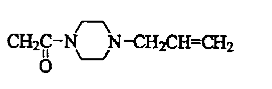 |
| CH=CH-CH=CH | 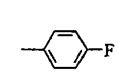 | 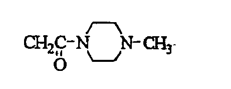 |

### BRIEF DESCRIPTION OF THE DRAWINGS

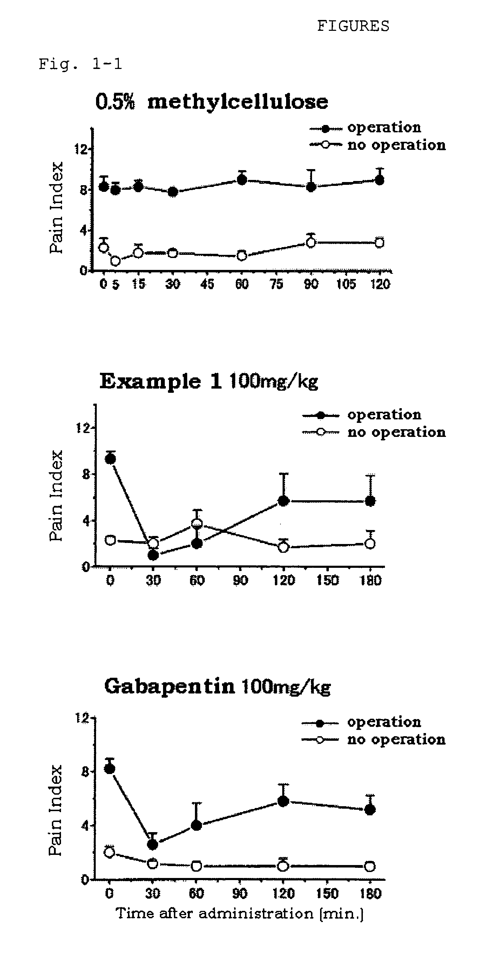

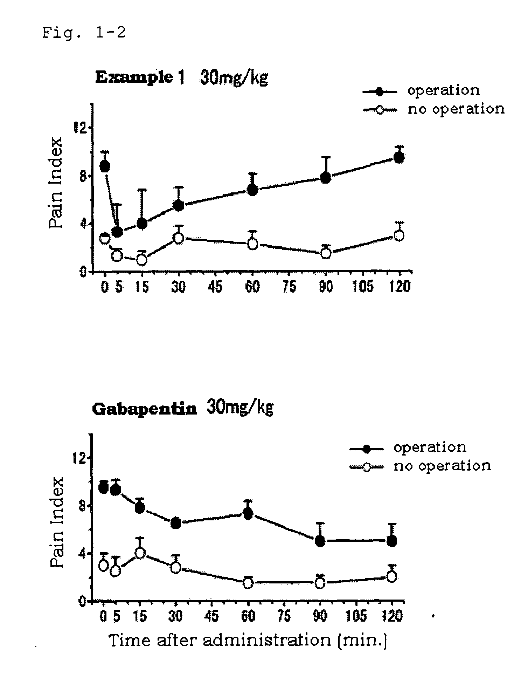

Fig. 1-1 is a graph depicting analgesic effect of the compound of example 1 and gabapentin (100mg/kg) in an animal model of neuropathic pain.
Fig. 1-2 is a graph depicting analgesic effect of the compound of example 1 and gabapentin (30mg/kg) in an animal model of neuropathic pain.
Fig. 2-1 is a graph depicting analgesic effects of the compounds of examples 1-6 in an animal model of neuropathic pain.
Fig. 2-2 is a graph depicting analgesic effects of the compounds of examples 7-14 in an animal model of neuropathic pain.

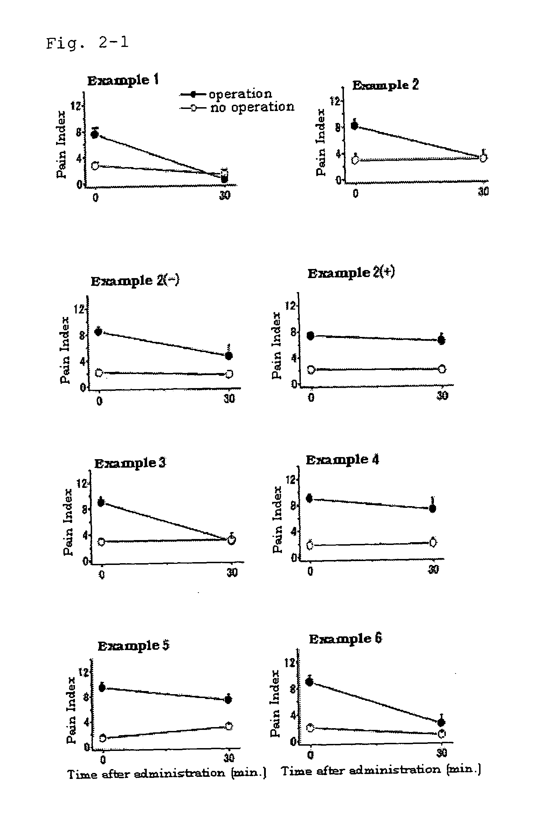

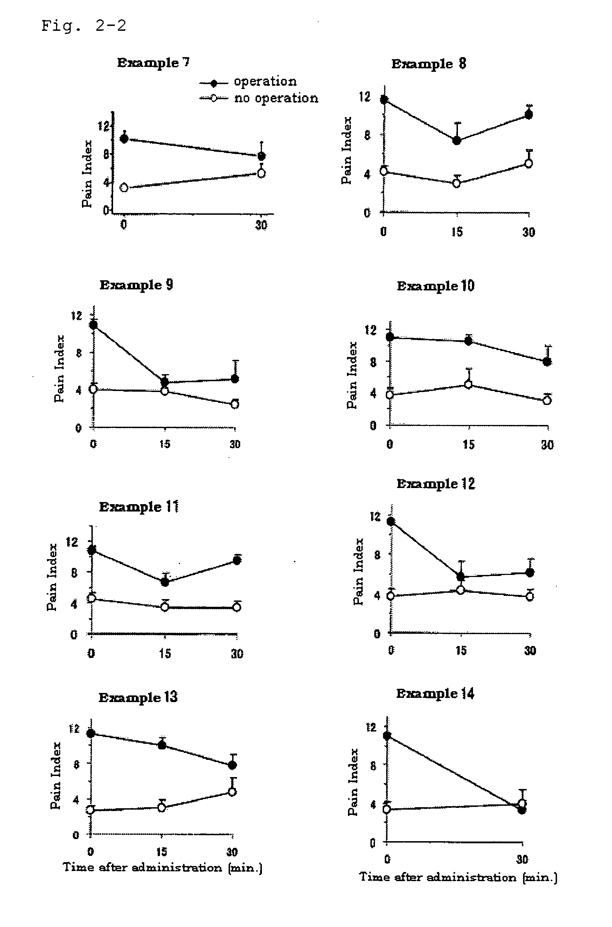

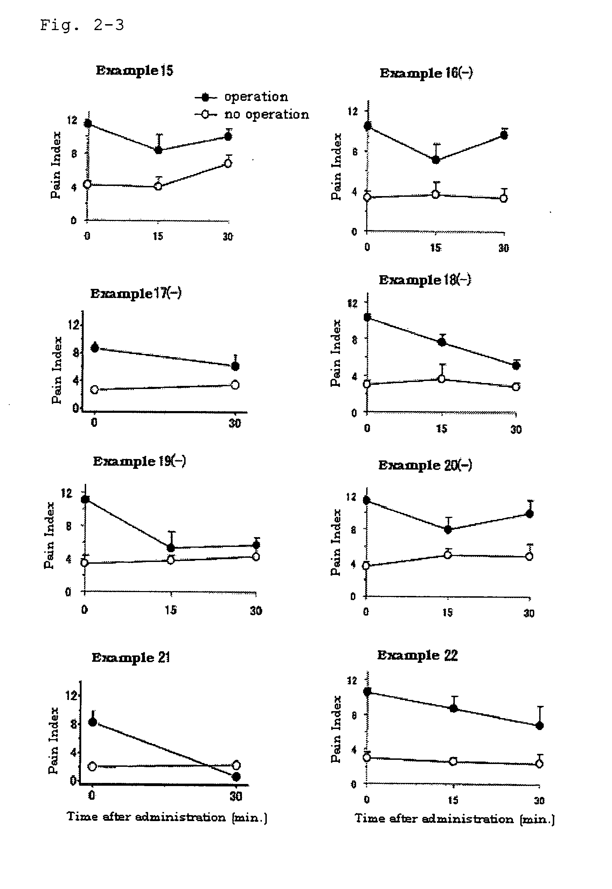

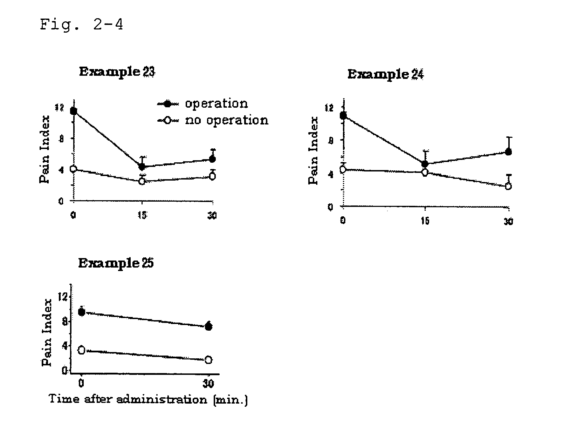

Fig. 2-3 is a graph depicting analgesic effects of the compounds of examples 15-22 in an animal model of neuropathic pain.
Fig. 2-4 is a graph depicting analgesic effects of the compounds of examples 23-25 in an animal model of neuropathic pain.

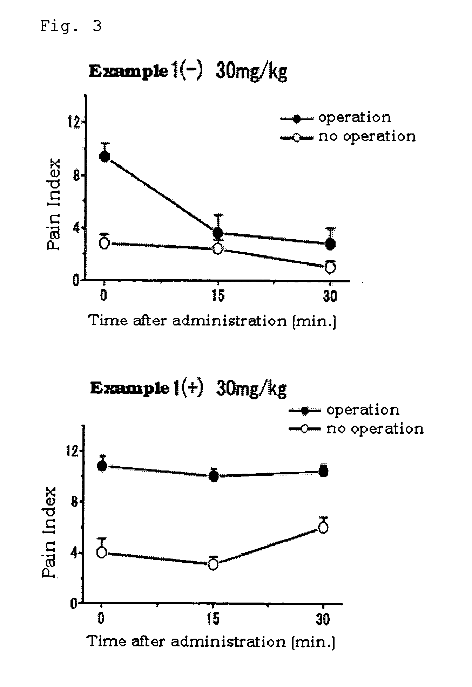

Fig. 3 is a graph depicting analgesic effect of the (+) and (-) isomers of the compound of example 1 in an animal model of neuropathic pain.

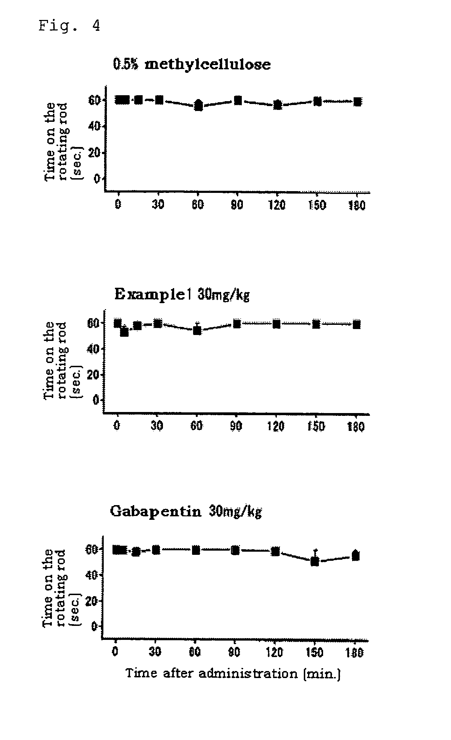

Fig. 4 is a graph depicting the effects of the compound of example 1 and gabapentin in the motor coordination test.

### BEST MODE FOR CARRYING OUT THE INVENTION

According to the present invention, R₁ and R₂ in formula (I) may be the same or different linear or branched C1-6 alkyls, preferably C1-3 alkyls and especially, both of R₁ and R₂ are methyl.

Alternatively, R₁ and R₂ may taken together form a condensed ring having conjugated double bonds, or they taken together form a moiety of -O-CH₂-O-, CH₂-O-CH₂- or - CH₂-CH₂-CH₂-.

X is halogen or C1-6 alkoxy, or forms together with the phenyl moiety to which the X is attached a group:

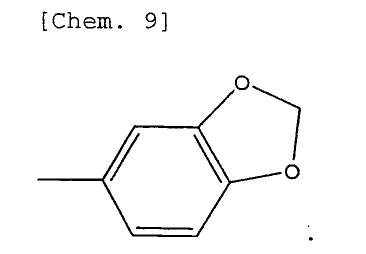

The halogen atom may be chlorine, fluorine, bromine or iodine, and chlorine or fluorine atom, especially fluorine atom, is preferable. When X is a halogen atom, m is an integer of 1 or 2. The halogen atom may be at the meta- and/or para- position of the phenyl moiety.

The alkoxy group may be C1-3 alkoxy and preferably, methoxy group. Especially, the compound having methoxy group at the para-position of the phenyl moiety.

When Y is 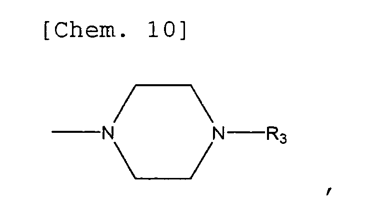 R₃ is linear or branched C1-6 alkyl, linear or branched C3-6 alkenyl, linear or branched C3-6 alkynyl, C5-6 cycloalkyl, a group of 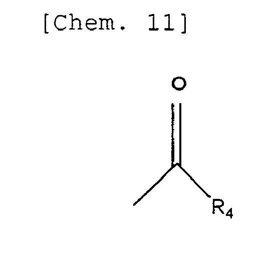 wherein R₄ is C1-4 alkyl,
or a group of

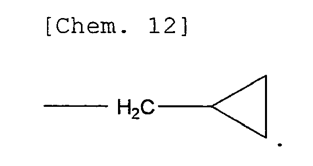

Among the above, preferred R₃ may include C1-6 alkyl, C3-6 linear alkenyl and C3-6 linear alkynyl; and linear C1-6 alkyl, -CH(CH₃)₂, -CH₂CH=CH₂, -CH₂-C≡CH, C(=O)CH₃, and a group of 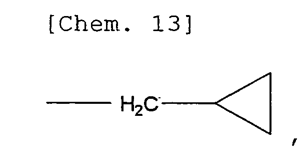 cyclopentyl and cyclohexyl groups are especially preferable.

Z is oxygen or sulfur atom and oxygen is more preferable.

The compound (I) of the present invention may be manufactured by any known method. For example, the compound of formula (I) may be manufactured by reacting compound (VII) obtained according to scheme 1 with a corresponding amine compound (VIII) in dimethylformamide or tetrahydrofuran in the presence of WSC [1-ethyl-3-(3-dimethylaminopropyl)carbodiimide hydrochloride] and HOBT (1-hydroxybenzotriazole hydrate). 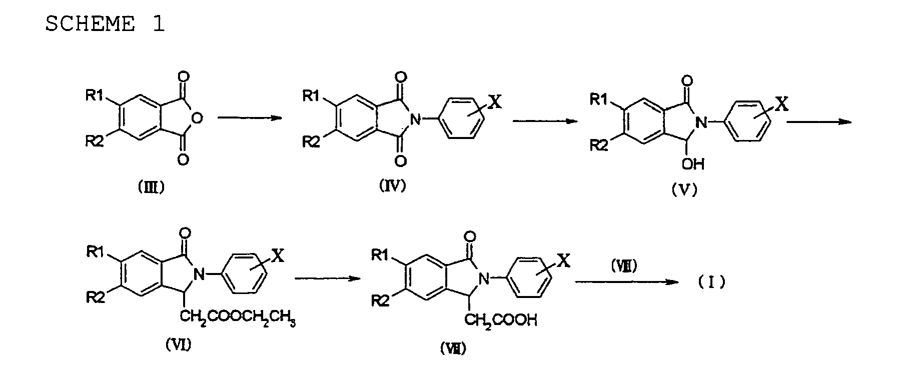 [In the above scheme, R₁, R₂ and X are as above defined].

The compound (III) used as a starting material in scheme 1 can be obtained as follows:
4,5-Dimethylphthalic anhydride (III-1) may be prepared by heating the acid anhydride, which is obtained by reacting 2,3-dimethyl-1,3-butadiene and maleic anhydride, in acetic acid together with bromine.
4,5-Diethylphthalic anhydride (III-2) may be prepared by converting the dicyano compound obtained according to J. Heterocyclic Chem., 22, 575 (1985) into the corresponding dicarboxylic acid with sulfuric acid followed by dehydrating (cyclizing) with acetic anhydride.

5,6-Indandicarboxylic anhydride (III-3) may be prepared by reacting 1,6-heptadiyne and diethyl acetylenedicarboxylate to give the diester compound, converting the diester compound into dicarboxylic acid compound with hydrochloric acid followed by dehydrating (cyclizing) with acetic anhydride.

1,3-dihydro-2-benzofuran-5,6-dicarboxylic anhydride may be prepared in the same manner as (III-3) using propargyl ether as starting material.

1,3-Benzodioxole-5,6-dicarboxylic anhydride can be obtained from 1,2-dibromo-4,5-(methylenedioxy)benzene in the same manner as compound (III-2).

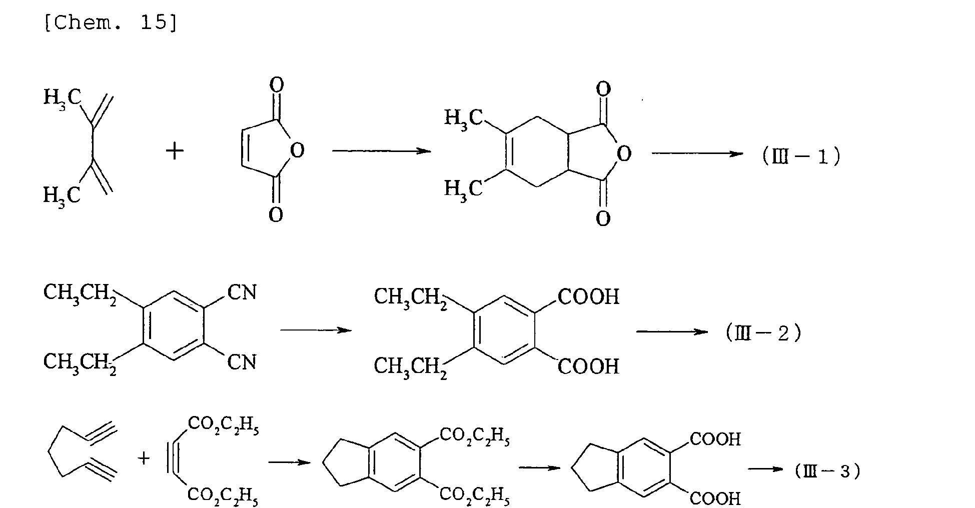

Thus obtained appropriate starting compound (III) is heated in acetic acid or dimethyl formamide with an amine compound of formula: 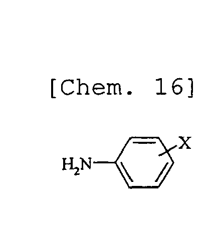 wherein X is as defined above to give the compound (IV). According to the method described in Japanese Patent Application Laid Open No. 58-189163, the compound (IV) is then reduced by sodium borohydride in a methanol and tetrahydrofuran mixed solution to give compound (V), and the compound (V) in toluene is heated with Ph₃P=CHCOOCH₂CH₃ and then hydrolyzed to give compound (VII).

Among the amine compounds of (VIII), piperazine derivative of the formula: 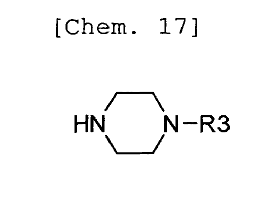 wherein R₃ is as defined above is commercially available or may be prepared according to Scheme 2 below.

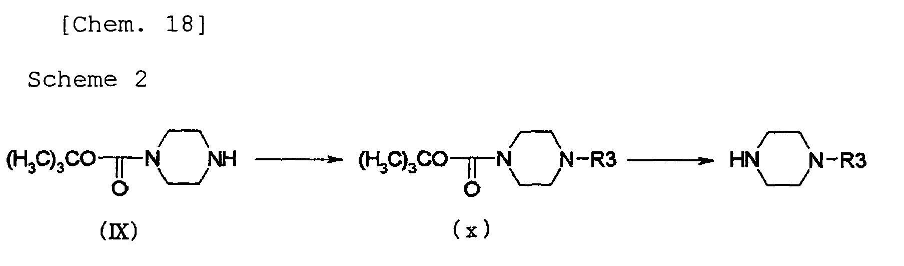

That is, by heating 1-tert-butoxycarbonyl piperazine (IX) with an appropriate bromo- or chloro-compound in acetonitrile in the presence of potassium carbonate to give compound (X) and the compound (X) is reacted with trifluoroacetic acid to give the desired piperazine derivative.

The compound of formula (I) may be contained in the composition in the form of salt and the composition comprising a salt of the compound of formula (I) is also within scope of the invention. As for salts, any pharmaceutically acceptable inorganic and organic salts may be employed. When the compound of formula (I) is basic, the salt may be an acid addition salt, for example salts with inorganic acids such as hydrochloride, sulfate, nitrate, phosphate and hydrobromide, and salts with organic acids such as acetate, propionate, fumarate, maleate, tartrate, citrate, malate, oxalate, benzoate, methanesulfonate and benzenesulfonate.

The compound of formula (I) may have optical isomers and the scope of the invention covers both isomers as well as the racemic compound. Usually, the compound of the present invention is obtained as racemic and the racemic compound may be divided into the optical isomers in a conventional manner known to the art. Among the optical isomers of the compound represented by formula (I), (-) isomer is more preferable.

The isoindoline derivative of formula (I) has an effect to suppress neuropathic pain in mammals and may preferably be used for treating or controlling neuropathic pain in a patient. The pharmaceutical composition of the invention can induce an analgesic effect with a lower dose than gabapentin that has been clinically used for treating neuropathic pain. In addition, the composition quickly provides the analgesic effect.

The pharmaceutical composition for controlling neuropathic pain of the present invention may be administered orally or parenterally, for example intravenously, epidurally, spinally, subcutaneously or intramuscularly to a mammal such as a human. The dosage form is not limited and may be designed appropriately by those skilled in the art. Examples of the dosage forms of the composition may include oral preparations including tablet, capsule such as soft capsule or microcapsule, granule, powder, syrup, emulsion and dispersion, and parenteral preparations including injectable solution, external preparations such as ointment and cream, suppositories such as rectal suppository and vaginal suppository, pellets, infusions, and sustained release formulation. The dosage forms may be administered orally or parenterally.

The pharmaceutical composition may be manufactured according to a conventional drug formulating method such as a method disclosed in Japanese Pharmacopoeia. The amount of the compound of the present invention in the pharmaceutical composition may be determined based on the formulation or dose and for example, may be about 0.1-100 weight%.

The composition of the present invention can be obtained by mixing the compound of formula (I) or a salt thereof with a pharmaceutically acceptable carrier or the like. The pharmaceutically acceptable carriers used in the present invention are conventional organic or inorganic carrier materials used in known pharmaceutical preparations, and those formulated as excipients, lubricants, binders, disintegrators, solvents, solubilizers, suspending agents, isotonizing agents, buffers and soothing agents may be used with no specific limitation. If necessary, pharmaceutical additives such as preservatives, antioxidants, coloring agents and sweeteners can also be added.

Preferable examples of the excipients include lactose, white sugar, D-mannitol, D-sorbitol, starch, α-starch, dextrin, crystalline cellulose, low-substituted hydroxypropyl cellulose, sodium carboxymethylcellulose, gum arabic, pullulan, light silicic anhydride, synthetic aluminum silicate and magnesium metasilicate aluminate. Preferable examples of the lubricants include magnesium stearate, calcium stearate, talc and colloidal silica.

Preferable examples of the binders include α-starch, sucrose, gelatin, gum arabic, methylcellulose, carboxymethyl cellulose, sodium carboxymethyl cellulose, crystalline cellulose, white sugar, D-mannitol, trehalose, dextrin, pullulan, hydroxypropyl cellulose, hydroxypropylmethyl cellulose and polyvinyl pyrrolidone.

Preferable examples of the disintegrators include lactose, white sugar, starch, carboxymethyl cellulose, calcium carboxymethyl cellulose, croscarmellose sodium, sodium carboxymethyl starch, light silicic anhydride and low-substituted hydroxypropyl cellulose.

Preferable examples of the solvents include injectable water, physiological saline, Ringer's solution, alcohol, propylene glycol, polyethylene glycol, sesame oil, corn oil, olive oil and cottonseed oil. Preferable examples of the solubilizers may include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferable examples of the suspending agents include surfactants such as stearyl triethanolamine, sodium laurylsulfate, laurylaminopropionic acid, lecithin, benzalconium chloride, benzetonium chloride and glycerine monostearate; hydrophilic polymers such as polyvinyl alcohol, polyvinyl pyrrolidone, sodium carboxymethyl cellulose, methyl cellulose, hydroxymethylcellulose, hydroxyethylcellulose, and hydroxypropylcellulose; polysorbates, and polyoxyethylene hardened sesame oil.

Preferable examples of the isotonizing agents include sodium chloride, glycerine, D-mannitol, D-sorbitol and glucose. Preferable examples of the buffers include buffers of phosphates, acetates, carbonates and citrates. Preferable examples of the soothing agents include benzyl alcohol and the like. Preferable examples of the preservatives include paraoxybenzoates, chlorobutanol, benzyl alcohol, phenethyl alcohol, dehydroacetic acid, and sorbic acid. Preferable examples of the antioxidants include sulfites and ascorbates. Preferable examples of the coloring agents include edible pigment, water-insoluble lake pigments and natural pigments (e.g., β-carotene, chlorophyll and red iron oxide). Preferable examples of the sweeteners include sodium saccharine, dipoptassium glycyrrhizinate, aspartame and stevia.

The composition for controlling neuropathic pain of the present invention may further comprise other pharmaceutically active ingredient as long as it impair the object of the instant invention.

The composition of the invention can be used for treating a patient suffering from neuropathic pain, for example herpes zoster acute pain, neuralgia after herpes zoster, neuralgia associated with diabetes, prolonged postoperative pain, reflex sympathetic dystrophy (RSD), RSD after tooth extraction, phantom limb pain and cancer pain. In the specification and claims of the instant application, the phrase "controlling pain" or "pain control" refers not only to control acute pain by administering the agent to a patient when suffered from an attack of pain or chronic pain by administering the same periodically to a patient suffered from chronic pain, but also the prophylactic use to prevent pain attack by administering the same to a patient an attack of pain is expected or to prevent repeated pain by periodically administering the same to a patient who is receiving pain attack repeatedly.

In the instant specification and claims, the "subject in need of pain control" covers the patients being suffered from pain as well as those expected to be suffered from pain.

The effective amount of the compound of formula (I) is not specifically limited and may be determined based on the age, sex, body weight and general condition of the subject to be treated and the severity of the neuropathic pain to be controlled.

Typically, but not limited thereto, when the composition is formulated as an oral analgesic, about 0.1-100mg/kg, preferably, 3.0-30.0mg/kg of the isoindoline compound represented by formula (I) may be administered orally to the subject. The onset of the action of the isoindoline derivative of the invention occurs rapidly and therefore, the administration may be made when the subject is received an acute pain. For treating chronic pain, the composition may be administered three or four times a day, for example at morning, daytime, evening and/or bedtime. If desired, the composition of the present invention may be administered in combination with an adjuvant analgesics such as anti-inflammatory, antidepressant and anticonvulsant agent.

The instant invention will be further illustrated with working examples shown below. However, the scope of the invention will not be limited to the examples.

### Synthetic Example 1

### 4,5-Diethylphthalic anhydride

### (a) 4,5-Diethylphthalic acid

1,2-dicyano-4,5-diethylbenzene (2.3g, 12mmol) was stirred with heating in 75% sulfuric acid (30 ml) at 150°C for 3.5 hrs. The reaction mixture was poured into ice-cold water. The precipitated crystals were collected by filtration, washed with water, and dissolved in 10% aqueous sodium hydroxide solution. The insoluble materials were separated by filtration, and the resulting filtrate was made acidic with concentrated hydrochloric acid. The precipitated crystals were collected by filtration, washed with water, and dried to give 1.5 g of 4,5-diethylphthalic acid.

### (b) 4,5-diethylphthalic anhydride

The product of the above (a) (1.5 g, 6.7 mmol) was heated under reflux in acetic anhydride (10 ml) for 1 hr. The reaction mixture was concentrated under reduced pressure, and the residue was dissolved in 10% aqueous sodium hydroxide. The insoluble materials were collected by filtration, washed with water, and dried to give 0.31 g of the title compound.

### Synthetic Example 2

### 4,5-dimethylphthalic anhydride

### (a) 5,6-dimethyl-3a,4,7,7a-tetrahydro-2-benzofuran-1,3-dione

To a solution of maleic anhydride (5.4 g, 55 mmol) in benzene (50 ml), 2,3-dimethyl-1,3-butadiene (6.3 ml, 55 mmol) was added dropwise and the mixture was stirred overnight at 25°C. After removing the insoluble materials by filtration, the filtrate was concentrated under reduced pressure to give 9.5 g of 5,6-dimethyl-3a,4,7,7a-tetrahydro-2-benzofuran-1,3-dione.

### (b) 4,5-dimethylphthalic anhydride

To a solution of the product of the (a) as above (9.5 g, 53 mmol) in acetic acid (28 ml), bromine (6.1 ml, 0.12 mol) in acetic acid (28 ml) was added dropwise at 115°C over a period of 45 minutes, and the mixture was heated under reflux for 1 hr. The reaction mixture was left overnight, and the precipitated crystals were collected by filtration, washed with diethyl ether, followed by drying to give 3.5 g of the title compound.

### Synthetic Example 3

### 5,6-indandicarboxylic anhydride

### (a) Diethyl 5,6-indandicarboxylate

Diethyl acetylenedicarboxylate (1.0 ml, 6.3 mmol) and dicarbonylcyclopentadienylcobalt (0.1 ml, 0.62 mmol) were added dropwise to a solution of 1,6-heptadiyne (0.72 ml, 6.3 mmol) in xylene (5 ml), and stirred at 80°C for 5 days. To the reaction mixture, dilute hydrochloric acid was added and the mixture was extracted with ethyl acetate. The organic layer was washed with saturated brine, dried and concentrated under reduced pressure, followed by purifying the residue by silica gel chromatography (chloroform, successively hexane : ethyl acetate = 10 : 1) to give 0.36 g of diethyl 5,6-indandicarboxylate.

### (b) 5,6-indandicarboxylic acid

To a solution of the product of the above (a) (0.36 g, 1.4 mmol) in acetic acid (0.8 ml) was added concentrated hydrochloric acid (0.4 ml) and stirred at 80°C overnight. To the reaction mixture was added ice-cold water, and the precipitated crystals were collected by filtration, washed with water, followed by drying to give 0.28 g of 5,6-indandicarboxylic acid.

### (c) 5,6-indandicarboxylic anhydride

The product of the above (b) (0.28 g, 1.4 mmol) was heated under reflux in acetic anhydride (6.7 ml) overnight. The reaction mixture was poured into ice-cold water, and the precipitated crystals were collected by filtration, washed with water, followed by drying to give 0.25 g of the title compound.

### Synthetic Example 4

### 1,3-dihydro-2-benzofuran-5,6-dicarboxylic anhydride

By using propargyl ether as starting material, the title compound was obtained in the same manner as Synthetic Example 3.

### Synthetic Example 5

### 1,3-benzodioxole-5,6-dicarboxylic anhydride

By using 1,2-dibromo-4,5-(methylenedioxy)benzene, the title compound was obtained according to the synthesis of 4,5-diethylphthalic anhydride.

### Synthetic Example 6

### 5,6-dimethyl-2-(3-fluorophenyl)-3-carboxymethylisoindolin-1-one [IUPAC name: 2-[2-(3-fluorophenyl)-5,6-dimethyl-3-oxo-2,3-dihydro-1H-isoindol-1-yl]acetic acid]

### (a) 2-(3-fulorophenyl)-5,6-dimethyl-1H-isoindol-1,3(2H)-dione

4,5-dimethylphthalic anhydride (4.0 g, 22.7 mmol) and 3-fluoroaniline (2.5 g, 22.7 mmol) in acetic acid (70mL) were heated under reflux for 2 hours. After cooling, the precipitated crystals were collected by filtration, washed with petroleum ether and dried to give 4.55 g of 2-(3-fluorophenyl)-5,6-dimethyl-1H-insoindole-1,3(2H)-dione.

¹H-NMR (CDCl₃) δ: 2.45 (6H, s, CH₃), 7.06-7.11 (1H, m, PhH), 7.22-7.29 (2H, m, PhH), 7.43-7.48 (1H, m, PhH), 7.71 (2H, s, C_{4,7}-H)

### (b) 2-(3-fluorophenyl)-3-hydroxy-5,6-dimethyl-1-isoindolinone

The product of the above (a) (4.55 g, 16.9 mmol) was suspended in methanol (70 ml) and tetrahydrofuran (70 ml), and sodium borohydride (1.28 g, 33.8 mmol) was added by portions thereto with stirring on ice, followed by stirring at the same temperature for 20 minutes. To the reaction mixture, water was added and the precipitated crystals were collected by filtration, washed with water and dried to give 3.25 g of 2-(3-fluorophenyl)-3-hydroxy-5,6-dimethyl-1-isoindolinone.

### (c) 2-[2-(3-fluorophenyl)-5,6-dimethyl-3-oxo-2,3-dihydro-1H-isoindol-1-yl] acetic acid

The product of the above (b) (3.25 g, 12 mmol) and (carboethoxymethylene)triphenylphosphorane (4.88 g, 14 mmol) in toluene (80 ml) were heated under reflux under an argon atmosphere for 3.5 hrs. The reaction mixture was concentrated under reduced pressure, the residue was dissolved in chloroform. The unreacted (carboethoxymethylene)triphenylphosphorane was removed by being adsorbed to silica gel. The crude product in mixed solvent of methanol (40ml) and 15% aqueous K₂CO₃ (11 ml) was heated for 4 hours at 80°C with stirring. The reaction mixture was concentrated under reduced pressure, water was added to the residue and the mixture was extracted with diethyl ether. The aqueous layer was obtained and acidified with concentrated hydrochloric acid. The precipitated crystals were collected by filtration, washed with water and dried to give 2.36g of the title compound.

### Synthethic Example 7

By using 5,6-dimethyl-2-substituted-isoindolin-1,3-dione as starting material, 5,6-dimethyl-3-carboxymethyl-2-substituted-isoindolin-1-one was obtained according to the same manner as Synthetic Example 6.

### Synthetic Example 8

### (-)-2-[2-(3-fluorophenyl)-5,6-dimethyl-3-oxo-2,3-dihydro-1H-isoindol-1-yl] acetic acid

Racemic 2-[2-(3-fluorophenyl)-5,6-dimethyl-3-oxo-2,3-dihydro-1H-isoindol-1-yl] acetic acid was reacted with (S)-(-)-phenylethylamine to form a salt, and the salt was subjected to fractional recrystallization using methanol. The resulting salt was treated with 1N hydrochloric acid to give the title compound.
specific optical rotation [α]²⁹_{D}=-61.6° (c=1.0, chloroform : methanol = 1:1)

### Synthetic Example 9

### 1-(2-propynyl)piperazine

### (a) 4-(2-propynyl)-1-piperazinecarboxylic acid tert-butyl ester

A mixture of 1-tert-butoxycarbonylpiperazine (500 mg, 2.68 mmol), 3-bromo-1-propine (200 mg, 2.68 mmol) and potassium carbonate (445 mg, 3.22 mmol) in acetonitrile (5ml) was stirred at 90°C for 2 hours. The reaction mixture was added with water and extracted with chloroform. The extract was concentrated under reduced pressure, and the residue was purified by silica gel chromatography (chloroform) to give 4-(2-propynyl)-1-piperazinecarboxylic acid tert-butyl ester.

¹H-NMR (CDCl₃) δ: 1.46 (9H, s, CH₃), 2.27 (1H, t, CH), 2.51 (4H, t, piperazine), 3.32 (2H, d, CH₂), 3.47 (4H, t, piperazine)

### (b) 1-(2-propynyl)piperazine

4-(2-propynyl)-1-piperazinecarboxylic acid tert-butyl ester (693 mg, 3.09 mmol) in trifluoroacetic acid (5ml) was stirred for 1.5 hours at the room temperature. The reaction mixture was concentrated under reduced pressure. The residue was added with water, the pH of the mixture was adjusted to about 10 with aqueous 1N-NaOH and then, the mixture was extracted with chloroform. The organic layer was obtained and concentrated under reduced pressure to give the title compound.

¹H-NMR (DMSO-d₆) δ: 2.25 (1H, t, CH), 2.54 (4H, b, piperazine), 2.93 (4H, t, piperazine), 3.29 (2H, d, CH₂)

### Synthetic Example 10

### 5,6-dimethyl-2-(3-fluorophenyl)-3-(4-methyl-1-piperazinyl)carbonylmethyl isoindolin-1-one

5,6-dimethyl-2-(3-fluorophenyl)-3-carboxymethyl isoindolin-1-one [IUPAC Name: 2-[2-(3-fluorophenyl)-5,6-dimethyl-3-oxo-2,3-dihydro-1H-isoindol-1-yl]acetatic acid] (0.50 g, 1.6 mmol), 1-methylpiperazine (0.16 g, 1.6 mmol), 1-ethyl-3-(3-dimethylaminopropyl)carbodiimide hydrochloride (0.31 g, 1.6 mmol) and 1-hydroxybenzotriazol hydrate (0.25 g, 1.6 mmol) in tetrahydrofuran (40 ml) were stirred at 25°C for 16 hours. The reaction mixture was concentrated under reduced pressure and the residue was purified with silica gel chromatography (chloroform : methanol=20:1) to give 0.56g of the title compound.

¹H-NMR (CDCl₃) δ: 2.16-2.26 (2H, m, piperazine), 2.27 (3H, s, NCH₃), 2.36 (3H, s, CH₃), 2.37 (3H, s, CH₃), 2.34-2.42 (2H, m, piperazine), 2.41 (1H, dd, CH₂), 2.91 (1H, dd, CH₂), 3.20-3.31 (2H, m, piperazine), 3.64-3.72 (2H, m, piperazine), 5.77 (1H, dd, CH), 6.88-6.93 (1H, m, PhH), 7.38 (1H, s, C₄-H), 7.35-7.42 (2H, m, PhH), 7.58-7.62 (1H, m, PhH), 7.68 (1H, s, C₇-H)

### Synthetic Example 11

The compounds shown in Table 1 were obtained in the same manner as Synthetic Examples 2, 6, 7, 8, 9 and 10.

**[Table 1-1]**

| | | | |
|---|---|---|---|
| 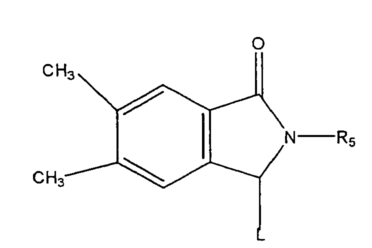 | | | |

| No. | R5 | L | melting point [°C] |
|---|---|---|---|
| Example 1 | 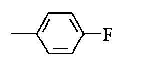 | 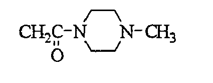 | 146-151 (1-hydrochloride salt) |
| Example 2 | 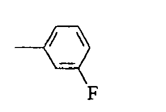 | 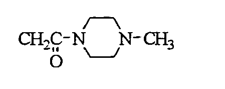 | 141-141.5 (1-hydrochloride salt) |
| Example 3 | 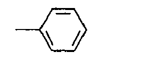 | 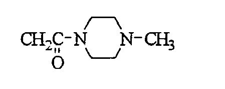 | 124-132 (1-hydrochloride salt) |
| Example 4 | 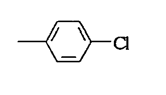 | 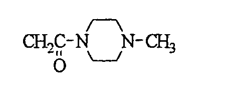 | 201.5-208.5 (1-hydrochloride salt) |
| Example 5 | 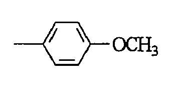 | 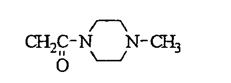 | decomposed, 226.5 (1-hydrochloride salt) |
| Example 6 | 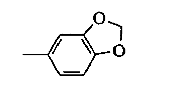 | 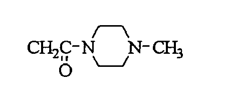 | 157.5-160.5 |
| Example 7 | 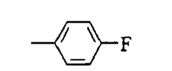 | 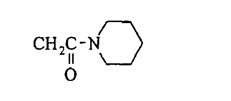 | 174-177 |
| Example 8 | 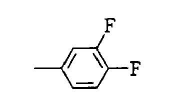 | 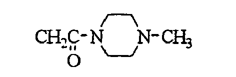 | 203.5-205 |
| Example 9 | 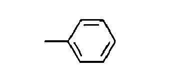 | 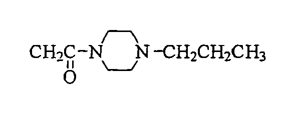 | 137-138 (1-hydrochloride salt) |

**[Table 1-2]**

| No. | R5 | L | melting point [°C] |
|---|---|---|---|
| Example 10 | 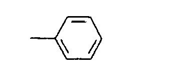 | 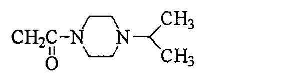 | 79-84 |
| Example 11 | 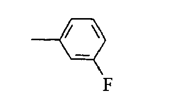 | 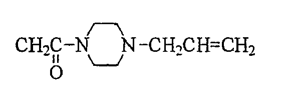 | 123-125 |
| Example 12 | 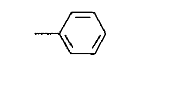 | 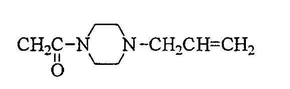 | 81-86 |
| Example 13 | 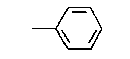 | 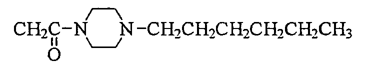 | 88-90 |
| Example 14 | 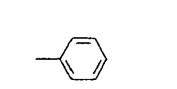 | 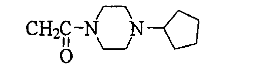 | 153-161 (1-hydrochloride salt) |
| Example 15 | 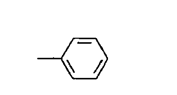 | 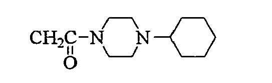 | 170-170.5 (1-hydrochloride salt) |
| Example 16 (-) | 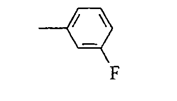 | 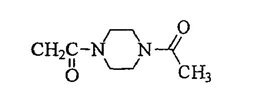 | 100-104 |
| Com. Ex. 1 | 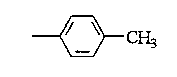 | 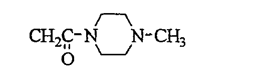 | 163.5-167 (1-hydrochloride salt) |
| Com. Ex. 2 | 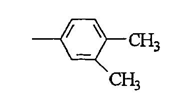 | 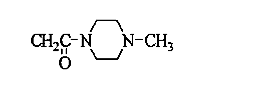 | 163.5-164 (1-hydrochloride salt) |
| Com. Ex. 3 | 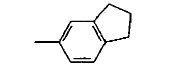 | 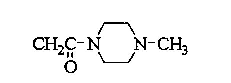 | 172-175 |
| Com. Ex. 4 | 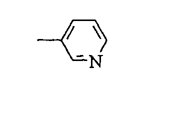 | 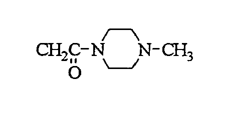 | 138-145 |

**[Table 1-3]**

| No. | R5 | L | melting point [°C] |
|---|---|---|---|
| Com. Ex. 5 | 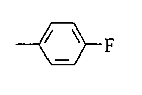 | 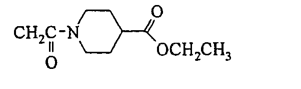 | 64-68 |
| Com. Ex. 6 | 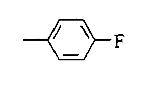 | 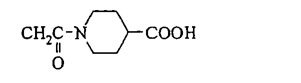 | 118.5-122.5 |
| Com. Ex. 7 | 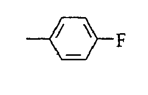 | 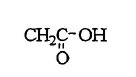 | 116-118 |
| Com. Ex. 8 | 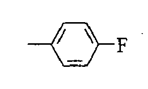 | 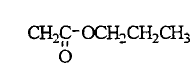 | 123-124 |
| Com. Ex. 9 | 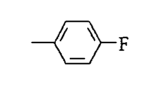 | 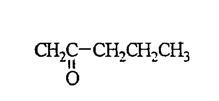 | 128-131 |
| Com. Ex. 10 | 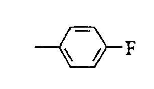 | 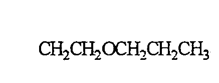 | 138-140 |
| Com. Ex. 11 | 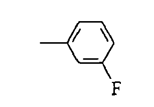 | 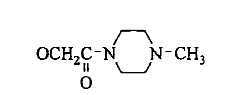 | 196-197 |
| Com. Ex. 12 | 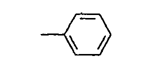 | 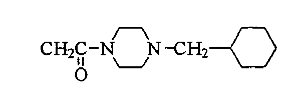 | 70-74 |
| Com. Ex. 13 | 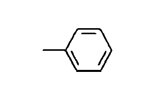 | 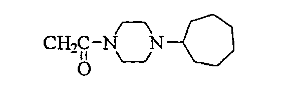 | 73-76 |
| Com. Ex. 14 | 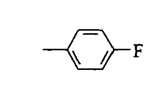 | 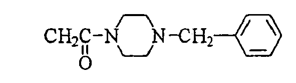 | 156.5-160 (1-hydrochloride salt) |

### Synthetic Example 12

### 2-[2-(3-fluorophenyl)-3-oxo-1,2,3,5,6,7-hexahydrocyclopenta [f]isoindol-1-yl] acetic acid

### (a) 2-(3-fluorophenyl)-6,7-dihydrocyclopenta[f]isoindol-1,3(2H,5H)-dione

5,6-indandicarboxylic anhydride (1.2 g, 6.4 mmol) and 3-fluoroaniline (708 mg, 6.4 mmol) in acetic acid (20 mL) were heated at 135 °C and stirred for 3 hours. The reaction mixture was concentrated under reduced pressure and the residue was purified with silica gel chromatography (chloroform) to give 1.57 g of 2-(3-fluorophenyl)-6,7-dihydrocyclopenta[f]isoindol-1,3(2H,5H)-dione.

¹H-NMR (CDCl₃) δ: 2.21 (2H, quintet, CH₂), 3.06 (4H, t, CH₂), 7.08 (1H, td, PhH), 7.22-7.29 (2H, m, PhH), 7.42-7.48 (1H, m, PhH), 7.75 (2H, s, C_{4,8}-H)

### (b) 2-(3-fluorophenyl)-3-hydroxy-3,5,6,7-tetrahydrocyclopenta[f]isoindol-1(2H)-one

The product of the above (a) (0.80 g, 2.8 mmol) was dispersed in a mixture of methanol (8 mL) and tetrahydrofuran (8 mL), and sodium borohydride (0.10 g, 2.8 mmol) was added by portions thereto with stirring on ice, followed by stirring at the same temperature for 30 minutes. To the reaction mixture, water was added and the precipitated crystals were collected by filtration, washed with water, and dried to give 0.79g of 2-(3-fluorophenyl)-3-hydroxy-3,5,6,7-tetrahydrocyclopenta[f]isoindol-1(2H)-one.

### (c) 2-[2-(3-fluorophenyl)-3-oxo-1,2,3,5,6,7-hexahydrocyclopenta[f]isoindol-1-yl] acetic acid

The product of the above (b) (1.6 g, 5.6 mmol) and (carboethoxymethylene)triphenylphosphorane (2.1 g, 6.2 mmol) in toluene (28 ml) were heated under reflux under an argon atmosphere for 3.0 hrs. The reaction mixture was concentrated under reduced pressure, the unreacted (carboethoxymethylene)triphenylphosphorane was removed by being adsorbed to silica gel. The crude product in mixed solvent of methanol (12ml) and 15% aqueous K₂CO₃ (4.6 ml) was heated for 3 hours at 80°C with stirring. The reaction mixture was concentrated under reduced pressure and water was added to the residue. The insoluble matters were removed by filtration. The filtrate was acidified with concentrated hydrochloric acid and the precipitated crystals were collected by filtration, washed with water and dried to give 1.8g of the title compound.

¹H-NMR (DMSO-d₆) δ: 2.16 (2H, quintet, CH₂), 2.55 (1H, dd, CH₂), 2.99 (4H, t, CH₂), 3.04 (1H, dd, CH₂), 5.49 (1H, dd, CH), 6.94 (1H, td, PhH ), 7.41(2H, s, C₄-H), 7.33-7.49 (3H, m, PhH), 7.74(2H, s, C₈-H)

### Synthetic Example 13

### (-)-2-[2-(3-fluorophenyl)-3-oxo-1,2,3,5,6,7-hexahydrocyclopenta[f]isoindol-1-yl]acetic acid

Racemic 2-[2-(3-fluorophenyl)-3-oxo-1,2,3,5,6,7-hexahydrocyclopenta[f]isoindol-1-yl] acetic acid and (S)-(-)-phenylethylamine were reacted to form a salt, and the salt was subjected to fractional recrystallization using ethanol. The resulting salt was treated with 1N hydrochloric acid to give the title compound. specific optical rotation [α]²⁹_{D} = -38.5 ° (c=1.0, methanol)

### Synthetic Example 14

The compounds shown in Table 2 were obtained in the same manner as Synthetic Examples 3, 4, 5, 9, 10, 12 and 13.

**[Table 2]**

| | | | | |
|---|---|---|---|---|
| 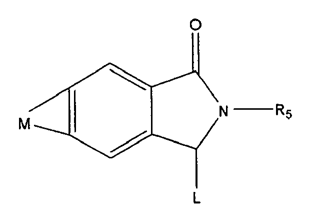 | | | | |

| No. | M | R5 | L | melting point[°C] |
|---|---|---|---|---|
| Example 17 (-) | CH₂CH₂CH₂ | 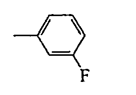 | 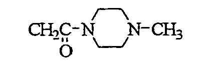 | 214-222 (1-hydrochloride salt) |
| Example 18 (-) | CH₂CH₂CH₂ | 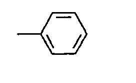 | 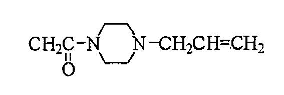 | 142-144 |
| Example 19 (-) | CH₂CH₂CH₂ | 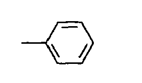 | 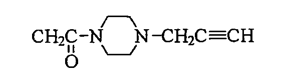 | 67-70 |
| Example 20 (-) | CH₂CH₂CH₂ | 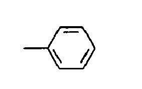 | 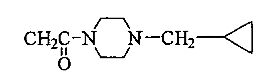 | 150-153 |
| Example 21 | OCH₂O | 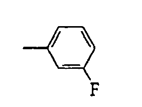 | 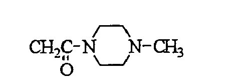 | 185.5-187.5 |
| Example 22 | OCH₂O | 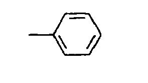 | 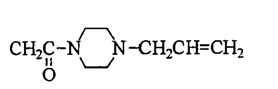 | 160-162.5 |
| Example 23 | CH₂OCH₂ | 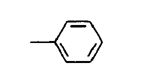 | 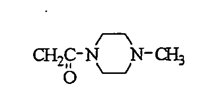 | 193.5-196 |
| Example 24 | CH₂OCH₂ | 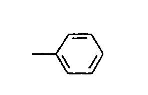 | 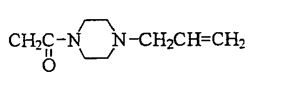 | 75-78 |
| Example 25 | CH=CH-CH=CH | 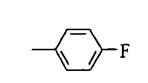 | 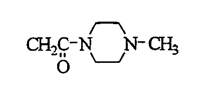 | 203-205.5 |

As far as the applicant knows, the compounds of examples 8, 11, 12, 13, 16, 18, 19, 20, 22, 23, 24 and 25 are novel.

### Pharmacological Test Example

The analgesic effects of the above-obtained compounds were evaluated using neuropathic pain model animal. As the negative control as well as vehicle, 0.5% aqueous methylcellulose was used. Gabapentin was used as a reference compound. The test compound in its free-form or hydrochloride salt was dissolved or dispersed in 0.5% aqueous methylcellulose and used.

The animal model of neuropathic pain was established according to the description of Pain, 87, 149(2000) with some modification. Briefly, the sciatic nerve of the right hind leg of a mouse under pentobarbital anesthesia was exposed. Among the three terminal branches of the sciatic nerve, tibial nerve was ligated tightly but the common peroneal and sural nerves were not. The left hind leg was used for non-operation control. One week after the ligation operation, each hind footpad of the animal was stimulated using von Frey filament (0.6g) for 6 times and the stimulation score was obtained at each stimulation based on the following criterion. All 6 stimulation scores were summed to give pain score. The maximum value of the pain score will be 12. According to the pain score thus obtained, the escape reaction against the stimulation was evaluated. Then, the test compound was orally administered to the animal and the escape reaction against the stimulation was evaluated according to the same manner as above.

In the von Frey test, the tip of von Frey filament was applied vertically to the center of the footpad while the filament was bent and the response of the mouse against the stimulation was evaluated. The evaluation criterion is as follows:
score 0: the mouse showed no reaction, score 1: the mouse moved gently the paw to escape from the stimulation and
score 2: the mouse licked the paw or aggressively moved the paw.

### Test Example 1

The analgesic effect of the compound of Example 1 on neuropathic pain was evaluated.
Three to five male BALB/c mice, 6-8 weeks old were used per group. The compound of Example 1 in an amount of 30 or 100mg/kg was administered orally to the animal. The pain score was evaluated with time, from before administration to 3 hours after administration. As reference compound, gabapentin in an amount of 30 or 100mg/kg was administered orally to the animal and the pain score was determined in the same manner. The result is shown in Figure 1.

As is shown in Figure 1, the analgesic effect of the compound of Example 1 was induced at lower doses than gabapentin. In addition, the onset of analgesic action occurred rapidly, i.e. after 5 minutes of 30mg/kg orally administration.

### Test Example 2

The analgesic effects of the compounds obtained in the above synthetic examples on neuropathic pain were evaluated.
Five to eight male BALB/c mice, 6-8 weeks old were used per group. Each compound in an amount of 30 mg/kg was administered orally to the animal. The pain score was evaluated before and 30 minutes after the administration. With respect to some compounds, the pain score was also evaluated 15 minutes after the administration. The results are shown in Figure 2.

As is apparent from Figure 2, the compounds of the invention have analgesic effect on neuropathic pain.

### Test Example 3

The (+) and (-) optical isomers of the compound of Example 1 were obtained by means of a conventional optical resolution of the compound. The analgesic effect of each compound on neuropathic pain was evaluated.
Six male BALB/c mice, 6-8 weeks old per group were used. Each compound in an amount of 30 mg/kg was administered orally to the animal. The pain score was evaluated before, and 15 and 30 minutes after the administration.

Results are shown in Figure 3. As is apparent from Figure 3, the (-) isomer exhibited analgesic effect but the (+) isomer did not.

The analgesic effects of the compounds evaluated in Test Examples 1-3 are summarized in Table 3. In the table below, compounds induced an analgesic effect equivalent to or higher than the compound of Example 1 were rated ++, those induced some analgesic effect but lower than that of the compound of Example 1 were rated + and compounds induced no analgesic effect were rated -.

**[Table 3]**

| Example | Analgesic Effect | Example | Analgesic Effect |
|---|---|---|---|
| Example 1 | + + | Com. Ex. 1 | - |
| Example 1 (-) | ++ | Com. Ex. 2 | - |
| Example 1 (+) | - | Com. Ex. 3 | - |
| Example 2 | ++ | Com. Ex. 4 | - |
| Example 2(-) | + | Com. Ex. 5 | - |
| Example 2 (+) | - | Com. Ex. 6 | - |
| Example 3 | ++ | Com. Ex. 7 | - |
| Example 4 | + | Com. Ex. 8 | - |
| Example 5 | + | Com. Ex. 9 | - |
| Example 6 | ++ | Com. Ex. 10 | - |
| Example 7 | + | Com. Ex. 11 | - |
| Example 8 | + | Com. Ex. 12 | - |
| Example 9 | ++ | Com. Ex. 13 | - |
| Example 10 | + | Com. Ex. 14 | - |
| Example 11 | + | | |
| Example 12 | ++ | | |
| Example 13 | + | | |
| Example 14 | + + | | |
| Example 15 | + | | |
| Example 16(-) | + | | |
| Example 17(-) | + | | |
| Example 18(-) | ++ | | |
| Example 19(-) | ++ | | |
| Example 20(-) | + | | |
| Example 21 | ++ | | |
| Example 22 | + | | |
| Example 23 | ++ | | |
| Example 24 | ++ | | |
| Example 25 | + | | |

### Motor Coordination Test

The effect of the compound of Example 1 on motor coordination was evaluated by means of mice rotating rod test.
Five male BALB/c, 6-7 weeks old mice per group were used. The mouse was trained for 2 or more days so that it could remain on the Rota-rod treadmill (Ugo Basile) in 3 cm in diameter rotating at 16rpm for at least 60 seconds when it was placed on the rod with the head directed against the direction of rotation. Before the evaluation, three trials were conducted with 10 min or more intervals and the mice which could remain on the rod more than 60 seconds in all three times were selected for the evaluation. The selected mice were divided into test groups.

The time obtained in the third trial was used as the before administration value. Mice were orally received 30mg/kg of the compound of Example 1 or gabapentin and placed on the rotating rod. The duration of time the mouse remained on the rod was recorded. A cut-off time of 60 seconds was employed and when an animal could remain more than 60 seconds, the time was recorded as 60 seconds. Result is shown in Figure 4.

As is apparent from Figure 4, no reduction of the remaining time were observed in both Example 1 and gabapentin groups. That is, the compound of the instant invention can induce the strong analgesic effect at a dosage (30mg/kg) which induces no affect on the motor coordination. In other word, the analgesic effect of the invention will be provided without deteriorating the motor function.

### Formulation Example

The ingredients were mixed so that the amount of the ingredient per tablet becomes as follows: 50 mg of the compound of Example 1, 200 mg of lactose, 40 mg of crystalline cellulose and 5 mg of magnesium stearate. The mixture is compressed with a tableting machine in a conventional manner to give tablet.

## Claims

1. A composition for controlling neuropathic pain, comprising a compound represented by formula (I): 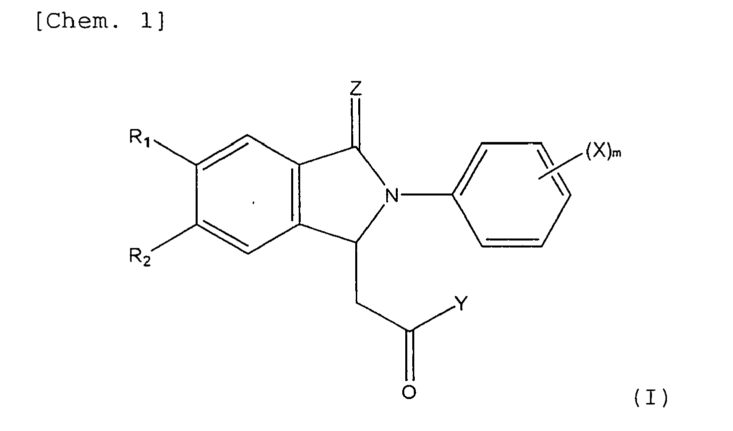 wherein R₁ and R₂ may be the same or different and represent C1-6 alkyl groups, or R₁ and R₂ taken together form 6-membered condensed ring having conjugated double bonds, or they taken together form a moiety of -O-CH₂-O-, -CH₂-CH₂-CH₂- or -CH₂-O-CH₂-;
X is halogen or C1-6 alkoxy, or taken together with the phenyl moiety to which the X is attached a group: 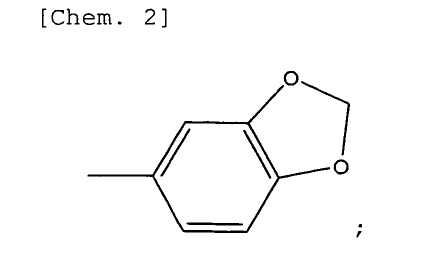 m is an integer of 0-2, provided that when X is C1-6 alkoxy, m is 0 or 1;
Y is 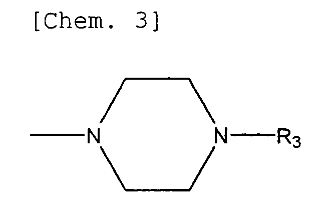 wherein R₃ is linear or branched C1-6 alkyl, linear or branched C3-6 alkenyl, linear or branched C3-6 alkynyl, C5-6 cycloalkyl, 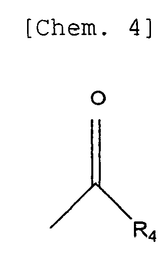 wherein R₄ is C1-4 alkyl, or 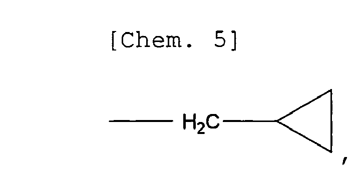 or 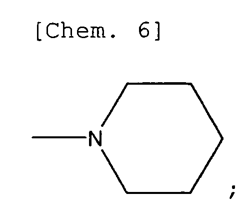 and
Z is oxygen or sulfur
or a salt thereof.

2. The composition of Claim 1, wherein both of R₁ and R₂ are methyl.

3. The composition of Claim 1, wherein R₁ and R₂ are taken together form a moiety of -O-CH₂-O-, -CH₂-CH₂-CH₂- or -CH₂-O-CH₂-.

4. The composition of any one of Claims 1-3, wherein X is halogen on the para- and/or meta-position of the phenyl moiety.

5. The composition of any one of Claims 1-3, wherein X is alkoxy on the para-position of the phenyl moiety.

6. The composition of any one of claims 1-3, wherein X is hydrogen or forms together with the phenyl moiety to which the X is attached a group:

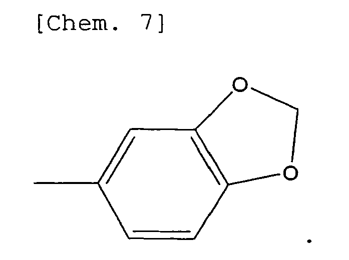

7. The composition of any one of Claims 1-6, wherein Y is 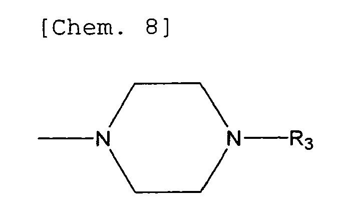 and R₃ is selected from the group consisting of linear C1-6 alkyl, -CH (CH₃)₂, -CH₂CH=CH₂, -CH₂-C≡CH, C(=O)CH₃, a group of 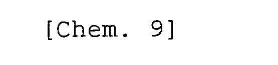 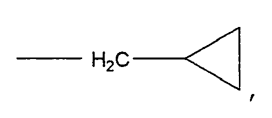 cyclopentyl and cyclohexyl.

8. The composition of any one of Claims 1-6, wherein Y is

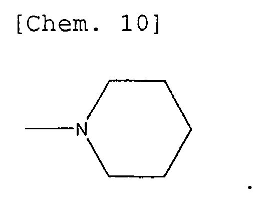

9. The composition of any one of Claims 1-8, wherein Z is oxygen.

10. The composition of Claim 1, wherein the compound of formula (I) is selected from the formulae shown below:
| | |
|---|---|
| 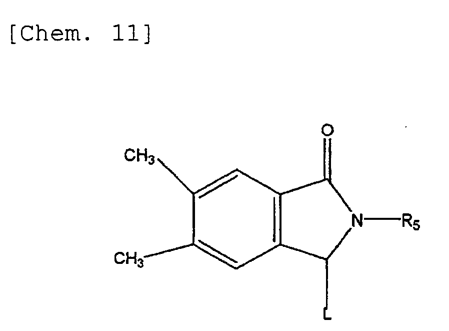 | |
| R5 | L |
|---|---|
| 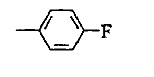 | 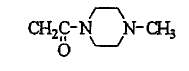 |
| 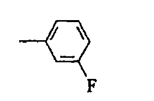 | 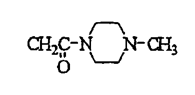 |
| 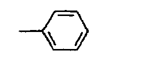 | 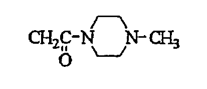 |
| 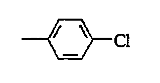 | 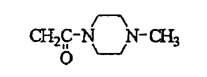 |
| 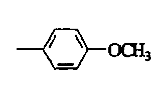 | 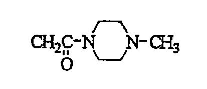 |
| 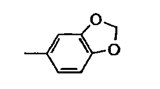 | 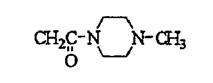 |
| 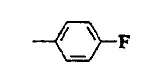 | 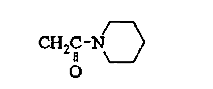 |
| 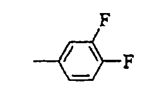 | 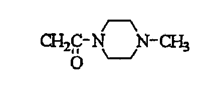 |
**[Chem. 12]**
| R5 | L |
|---|---|
| 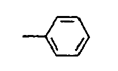 | 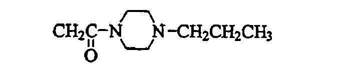 |
| 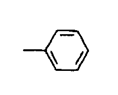 | 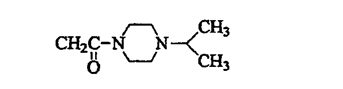 |
| 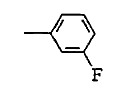 | 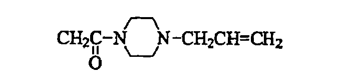 |
| 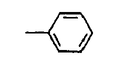 | 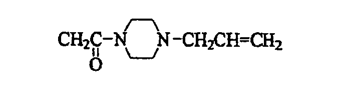 |
| 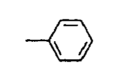 | 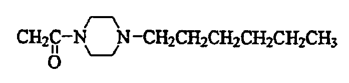 |
| 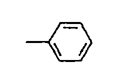 | 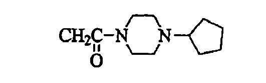 |
| 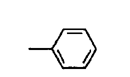 | 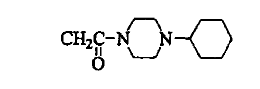 |
| 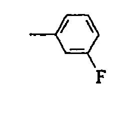 | 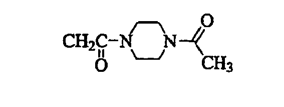 |

11. The composition of Claim 1, wherein the compound of formula (I) is selected from the formulae shown below:
| | | |
|---|---|---|
| 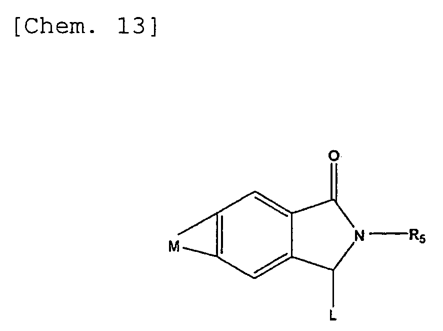 | | |
| M | R5 | L |
|---|---|---|
| CH₂CH₂CH₂ | 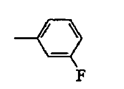 | 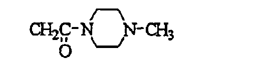 |
| CH₂CH₂CH₂ | 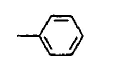 | 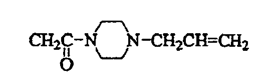 |
| CH₂CH₂CH₂ | 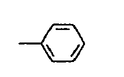 | 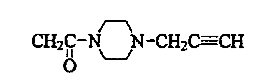 |
| CH₂CH₂CH₂ | 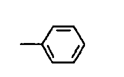 | 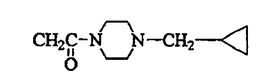 |
| OCH₂O | 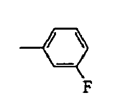 | 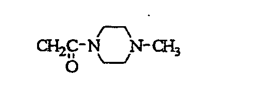 |
| OCH₂O | 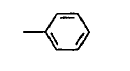 | 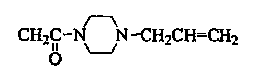 |
| CH₂OCH₂ | 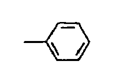 | 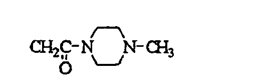 |
| CH₂OCH₂ | 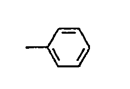 | 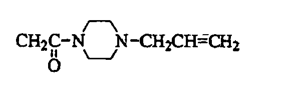 |
| CH=CH-CH=CH | 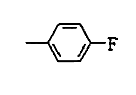 | 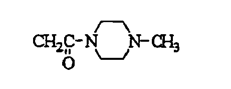 |

12. A method for controlling neuropathic pain which comprises administering an effective amount of the compound as defined in Claim 1 to a subject in need of pain control.

13. Use of the compound as defined in Claim 1 for manufacturing a pharmaceutical composition for controlling neuropathic pain.

14. A compound selected from the group consisting of:
| | |
|---|---|
| 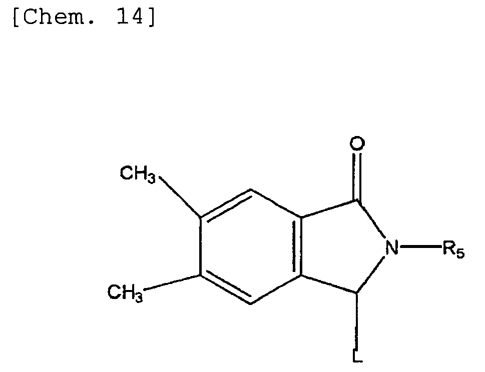 | |
| R5 | L |
|---|---|
| 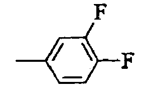 | 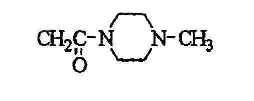 |
| 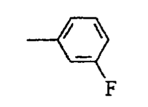 | 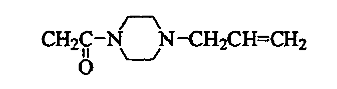 |
| 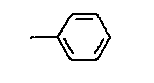 | 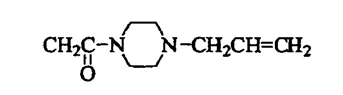 |
| 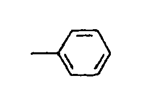 | 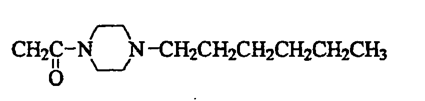 |
| 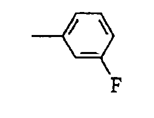 | 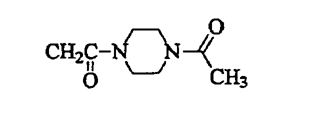 |
| | | |
|---|---|---|
| 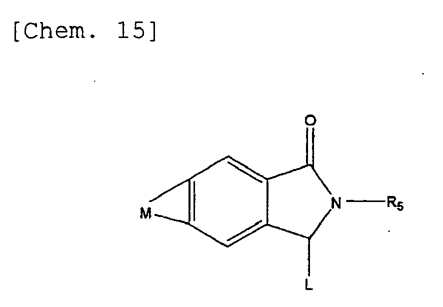 | | |
| M | R5 | L |
|---|---|---|
| CH₂CH₂CH₂ | 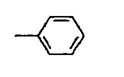 | 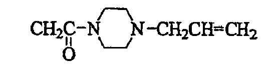 |
| CH₂CH₂CH₂ | 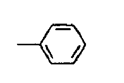 | 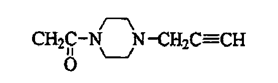 |
| CH₂CH₂CH₂ | 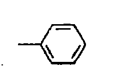 | 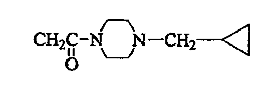 |
| OCH₂O | 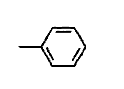 | 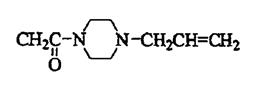 |
| CH₂OCH₂ | 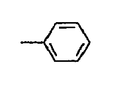 | 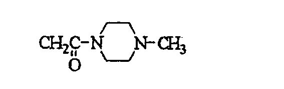 |
| CH₂OCH₂ | 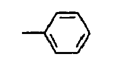 | 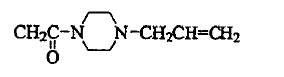 |
| CH=CH-CH=CH | 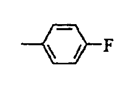 | 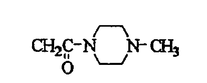 |

15. The compound of Claim 14, which is selected from the group consisting of:
| | |
|---|---|
| 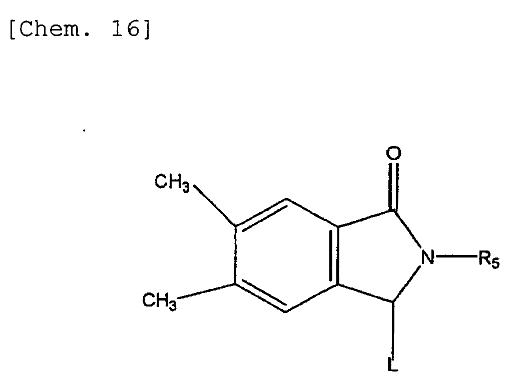 | |
| R5 | L |
|---|---|
| 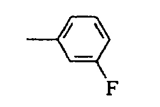 | 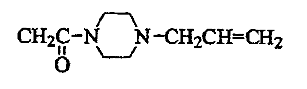 |
| 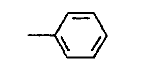 | 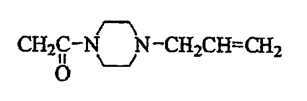 |
| 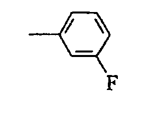 | 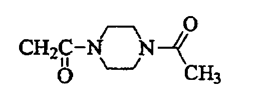 |
| | | |
|---|---|---|
| 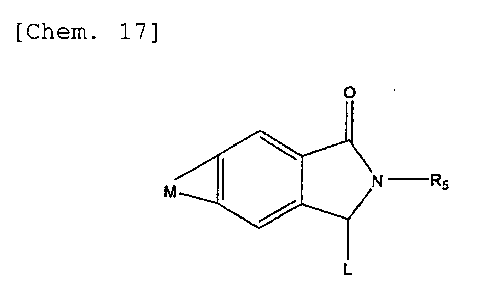 | | |
| M | R5 | L |
|---|---|---|
| CH₂CH₂CH₂ | 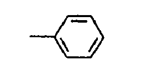 | 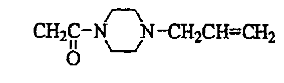 |
| CH₂CH₂CH₂ | 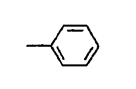 | 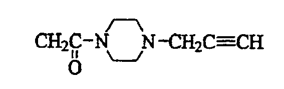 |
| CH₂CH₂CH₂ | 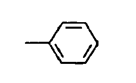 | 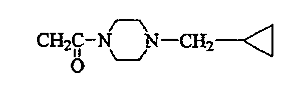 |
| OCH₂O | 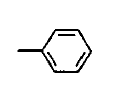 | 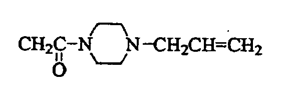 |
| CH₂OCH₂ | 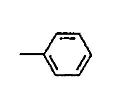 | 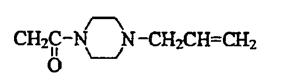 |
| CH=CH-CH=CH | 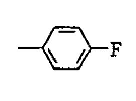 | 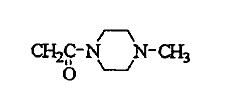 |
